# EUROPEAN PATENT APPLICATION

(11) **EP 3 932 372 A1**
(43) Date of publication of application: **05.01.2022**
(21) Application number: 21181927.1
(22) Date of filing: 28.06.2021
(51) Int. Cl.: A61F 2/958, A61F 2/966, A61F 2/01, A61M 25/10

(54) **ISOLATED STENTING WITH DISTAL SELF-EXPANDING OCCLUSION**

(30) Priority: 29.06.2020 US 202016914762
(71) Applicant: Neuravi Limited, Galway H91 K5YD (IE)
(72) Inventor: KEATING, Karl, Galway, H91 K5YD (IE); KELLY, Ronald, Galway, H91 K5YD (IE)
(74) Representative: Carpmaels & Ransford LLP

(57) **Abstract**

Catheter based systems for isolated stenting of an intravascular lesion can include two expandable occlusion devices with a self-expanding stent or a balloon expandable stent therebetween. Expandable occlusion devices can be expanded in a distal direction and a proximal direction in relation to the lesion to occlude vasculature. The stent can be deployed across a lesion while the occlusion devices are in place. One or both of the occlusion devices can include a fluid impermeable membrane to occlude blood flow. One or both of the occlusion devices and the stent can be surrounded by a sheath. The sheath can be retracted to allow one or both of the occlusion devices and the self-expanding stent to self-expand. Fragments dislodged from the lesion during stenting can be aspirated.

## Description

### FIELD OF INVENTION

The present invention generally relates to devices and methods for medical treatments, and more particularly, to devices and treatments for treating intravascular lesions such as lesions relating to intracranial atherosclerosis disease (ICAD).

### BACKGROUND

Atherosclerosis results from lesions which narrow and reduce the space in the lumen of vessels in the vasculature. Such lesions are usually composed of plaque, which can be fat, cholesterol, calcium, or other components of the blood. Severe occlusion or closure can impede the flow of oxygenated blood to different organs and parts of the body and result in disorders such as heart attack or stroke. Narrowing of vessels, or stenosis, increases the risk that clots and other emboli can lodge at such locations, especially in the neurovascular where vessel diameters are already small. Intracranial atherosclerotic disease ("ICAD") is the narrowing of those arteries and vessels supplying blood to the brain and represents the most common proximate mechanism of ischemic stroke.

Treatment for vascular occlusions can include utilizing drugs, such as anticoagulants or anti-platelet agents, as well as medical procedures such as surgical endarterectomy, angioplasty, and stenting. Much of the recent success in endovascular revascularization treatments (ERT) has been the further development of safe thrombectomy devices. Devices such as stentrievers, direct-aspiration systems, and other clot retrieval devices have been strongly associated with better clinical outcomes. However, these devices are primarily designed to recanalize the vessel by removing and retrieving an occluding embolus. Sufficient recanalization may not occur if there is also significant stenosis present at the occlusion site, increasing the need for implanted stents.

Treatment methods for addressing clots and lesions in the neurovascular in particular depend on the degree of stenosis, the shape of the target occlusion site (i.e. truncal, branching, etc.), and the patient's overall condition. For example, mechanical procedures often involve using medical devices to retrieve an occlusive clot and then utilizing balloons and stents to open a narrowed artery. Following the use of a stentriever or other clot retrieval device, a balloon is delivered to a target site and inflated to dilate the stenosis. The balloon can then be removed and exchanged through a catheter for a stent delivery device. A balloon can be inflated inside the stent to press the struts of the stent scaffold frame firmly against the inner wall of the vessel.

In the case of ICAD, prolonging treatment and/or crossing the occlusion with multiple devices can increase the likelihood that the ICAD ruptures or fragments. Such fragments can include but are not limited to blood clots, plaque, and other thrombi debris. The fragments can lead to vascular occlusions causing extensive stroke or death. Due to the difficulty in diagnosing ICAD, a physician may need to cross the lesion multiple times during a treatment, thereby further increasing the likelihood of rupture or fragmenting.

There therefore remains a need for systems and devices to continue to address and improve treatments for intravascular occlusions, specifically ICAD

### SUMMARY

It is an object of the present invention to provide catheter-based systems for isolated stenting of an intravascular lesion. Expandable occlusion devices can be expanded in a distal direction and a proximal direction in relation to the lesion to occlude vasculature. A stent can be deployed across the lesion while the occlusion devices are in place. Fragments dislodged during stenting can be aspirated.

An example treatment system can include a delivery tube, a distal expandable element, a self-expandable stent, and a sheath. The delivery tube can be sized to traverse vasculature. The distal expandable element can be disposed on the delivery tube and approximate a distal end of the delivery tube. The self-expandable stent can be disposed over a portion of the delivery tube in a proximal direction in relation to the distal expandable element. The sheath can surround the self-expandable stent. The sheath can further be positioned to inhibit the self-expandable stent from expanding and be moveable to allow the self-expandable stent to expand.

The treatment system can further include a guide catheter having a lumen therethrough sized to accommodate the delivery tube, the distal expandable element, the self-expanding stent, and the sheath.

The guide catheter can include a balloon guide catheter. The lumen of the balloon guide catheter can be sufficiently contiguous such that the lumen can be suitable for aspiration within vasculature.

The distal expandable element can include a balloon.

The distal expandable element can include a self-expandable portion. The sheath can surround the distal expandable element and be positioned to inhibit the distal expandable element from expanding. The sheath can further be moveable to allow the distal expandable element to expand.

The distal expandable element can be porous.

The intravascular system can further include a proximal expandable element disposed on the delivery tube in the proximal direction in relation to the stent.

The proximal expandable element can be porous.

The intravascular system can further include an aspiration catheter having a lumen therethrough sized to receive the delivery tube.

Another example intravascular treatment system can include a delivery tube, an expandable distal occluding element, a stent, a self-expandable proximal occluding element, and a sheath. The delivery tube can be sized to traverse vasculature. The expandable distal occluding element can be disposed on the delivery tube and approximate a distal end of the delivery tube. The stent can be disposed over the delivery tube in a proximal direction in relation to the distal occluding element. The system can further include an angioplasty balloon disposed on the delivery tube in the proximal direction in relation to the distal occluding element, and the stent can be disposed over the angioplasty balloon. The self-expandable proximal occluding element can be disposed on the delivery tube in the proximal direction in relation to the stent. The sheath can surround the proximal occluding element. The sheath can be positioned to inhibit the proximal occluding element form expanding and be moveable to allow the proximal occluding element to expand.

The sheath can be moveable to collapse the proximal occluding element after being moved to expand the proximal occluding element.

The proximal occluding element can be porous.

An example method for treating an intravascular lesion can include one or more of the following steps presented in no particular order. The method can further include additional steps as appreciated and understood by a person of ordinary skill in the art according to the teachings of this disclosure.

The method can include selecting a treatment system having a delivery tube, a distal expandable element, a self-expandable stent, and a sheath. The distal expandable element and stent can be disposed on the delivery tube and the sheath can be disposed over the stent. The sheath can be positioned to inhibit the stent from expanding.

The method can include positioning the treatment system across the lesion such that the distal expandable element is in the distal direction in relation to the lesion and the stent crosses the lesion.

The method can include expanding the distal expandable element.

The method can include moving the sheath, thereby allowing the stent to expand into the lesion.

The method can include selecting a guide catheter having an expandable element thereon and lumen therethrough. The lumen can be sized to accommodate the delivery tube, the distal expandable element, the self-expanding stent, and the sheath.

The method can include delivering the delivery tube through vasculature, within the balloon guide catheter.

The method can include positioning the proximal expandable element in a proximal direction in relation to the lesion.

The method can include expanding the proximal expandable element to circumferentially appose a blood vessel wall. The proximal expandable element can be expanded by inflation, self-expansion, mechanical expansion, or known means.

The method can include aspirating through the lumen of the guide catheter.

The method can include first expanding the distal expandable element, second expanding the proximal expandable element, and third moving the sheath, thereby allowing the stent to expand into the lesion.

The method can include first expanding the proximal expandable element, second expanding the distal expandable element, and third moving the sheath, thereby allowing the stent to expand into the lesion.

Selecting a treatment system can further include selecting the treatment system such that the sheath is disposed over the distal expandable element, the distal expandable element is self-expandable, and the sheath is positioned to inhibit the distal expandable element from expanding. Expanding the distal expandable element can include moving the sheath to allow the distal expandable element to expand.

The method can further include moving the sheath over the distal expandable element to collapse the distal expandable element.

Expanding the distal expandable element can include inflating the distal expandable element. When the distal expandable element is inflated, the method can further include deflating the distal expandable element.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1A is an illustration of an intravascular treatment system during a treatment step according to aspects of the present invention;
FIG. 1B is a cross-section view of the intravascular treatment system in FIG. 1A according to aspects of the present invention;
FIG. 1C is an additional cross-section view of the intravascular treatment system in FIG. 1A according to aspects of the present invention;
FIGs. 2A through 2F are a sequence of illustrations depicting steps of treatment of an intravascular lesion using the treatment system illustrated in FIG. 1A according to aspects of the present invention;
FIG. 3A is an illustration of another intravascular treatment system during a treatment step according to aspects of the present invention;
FIG. 3B is a cross-section view of the intravascular treatment system in FIG. 3A according to aspects of the present invention;
FIG. 3C is an additional cross-section view of the intravascular treatment system in FIG. 3A according to aspects of the present invention;
FIGs. 4A through 4N are a sequence of illustrations depicting steps of treatment of an intravascular lesion using the treatment system illustrated in FIG. 3A according to aspects of the present invention;
FIGs. 5A through 5E are a sequence of illustrations depicting steps of treatment of an intravascular lesion at a bifurcation using the treatment system illustrated in FIG. 3A according to aspects of the present invention;
FIGs. 6A and 6D through 6F are a sequence of illustrations depicting steps of treatment of an intravascular lesion using another treatment system including an angioplasty balloon according to aspects of the present invention;
FIGs. 6B and 6C are cross-sectional views of the intravascular treatment system as indicated in FIG. 6A;
FIGs. 7A and 7D through 7H are a sequence of illustrations depicting steps of treatment of an intravascular lesion using another treatment system according to aspects of the present invention;
FIGs. 7B and 7C are cross-sectional views of the intravascular treatment system as indicated in FIG. 7A;
FIG. 8 is a flow diagram outlining steps for treating an intravascular lesion according to aspects of the present invention; and
FIG. 9 is another flow diagram outlining steps for treating an intravascular lesion using the treatment system depicted in FIGs. 6A-6F according to aspects of the present invention.

### DETAILED DESCRIPTION

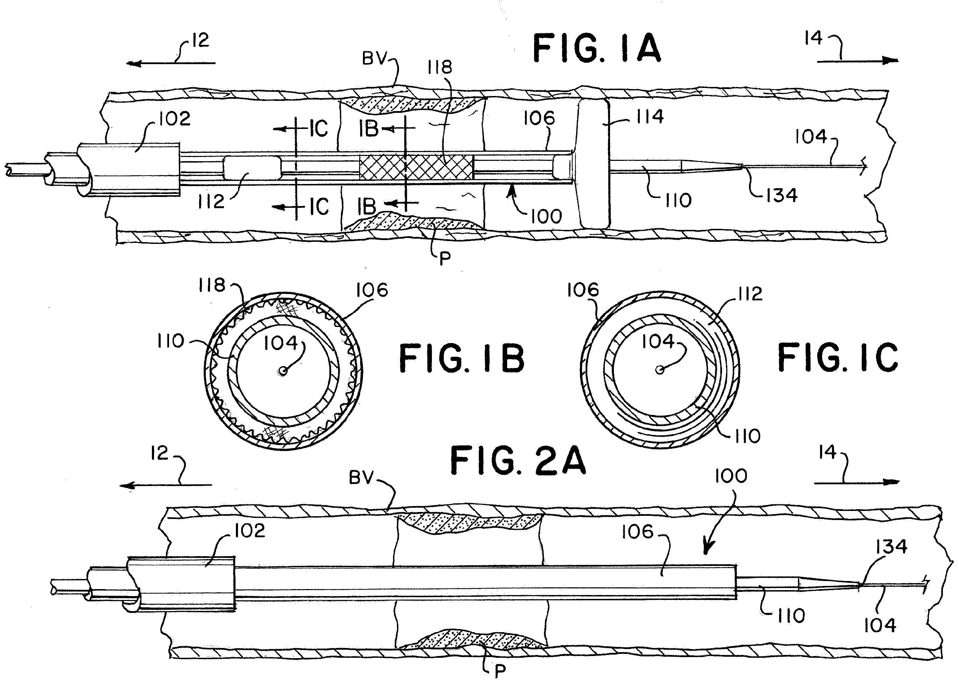

FIG. 1A is an illustration of an example intravascular treatment system 100 during treatment of a lesion P in a blood vessel BV. The treatment system 100 can include a delivery tube 110 sized to traverse a catheter 102, a distal expandable element 114, a proximal expandable element 112, and a stent 118. The system 100 can be sized to traverse vasculature BV to lesion P.

The delivery tube 110 can be sufficiently long such that a proximal end of the delivery tube 110 can be positioned outside the patient when the treatment system 100 is positioned at the lesion P. Configured as such, a user (e.g. physician) may manipulate a proximal end of the delivery tube 110. The system 100 can be delivered over a guide wire 104.

When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

The distal expandable element 114 can be disposed on the delivery tube approximate a distal end 134 of the delivery tube 110. The proximal expandable element 112 can be disposed on the delivery tube 110 in a proximal direction 12 in relation to the distal expandable element 114. The stent 118 can be disposed over the delivery tube 110 between the distal expandable element 114 and the proximal expandable element 112. The proximal expandable element 112 and the distal expandable element 114 can include a self-expandable, inflatable, mechanically expandable, and/or otherwise expandable element. The stent 118 can be self-expandable, expandable by a balloon, mechanically expandable, and/or otherwise expandable. When each of the distal expandable element 114, the stent 118, and the proximal expandable element 112 are self-expanding, the sheath 106 can be positioned over the proximal expandable element 112, the distal expandable element 114, and the stent 118 to inhibit those components 112, 114, 118 from expanding. By retracting the sheath 106 in the proximal direction 12, the distal expandable element 114, the stent 118, and the proximal expandable element 112 can self-expand in that order.

FIGs. 1B and 1C illustrate cross-sections of the intravascular treatment system 100 as indicated in FIG. 1A. FIG. 1B illustrates a cross-section view of the intravascular treatment system 100 at the stent 118. FIG. 1C illustrates a cross-section view of a proximal portion of the intravascular treatment system 100 relative to the stent 118. In FIGs. 1B and 1C, the delivery tube 110 can be delivered over the guide wire 104 to the desired location. FIG. 1B illustrates the sheath 106 can be positioned over the stent 118 disposed over the delivery tube 110 to prevent the stent 118 from self-expanding until desired. FIG. 1C illustrates the sheath 106 can be positioned over the proximal expandable element 112 to prevent the proximal expandable element 112 from self-expanding until desired.

FIGs. 2A through 2F illustrate a sequence of treatment steps using system 100. In FIG. 2A, the delivery tube 110, while surrounded by sheath 106, can be positioned across the lesion P within the blood vessel BV. To reach the position illustrated in FIG. 2A, first a guide wire 104 can be extended through vasculature of the patient and positioned across the lesion P, then the catheter 102 and the delivery tube 110 can be translated in the distal direction 14 over the guide wire 104 to the illustrated position. The sheath 106 can surround the delivery tube 110 such that the sheath 106 covers the distal expandable element 114, the stent 118, and the proximal expandable element 112. The catheter 102 need not cross the lesion P but can cross the lesion P if appropriately sized to minimize likelihood of dislodging fragments from the lesion P during crossing.

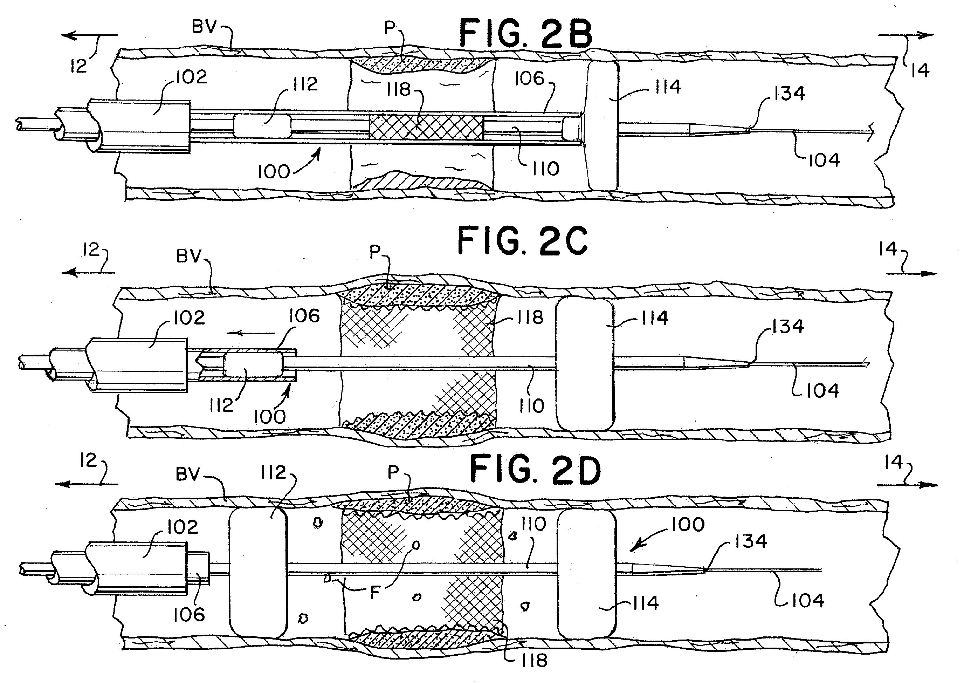

In FIG. 2B, the sheath 106 can be retracted in the proximal direction 12, exposing the distal end 134 of the delivery tube 110 including the distal expandable element 114. As the sheath 106 is retracted, the distal expandable element 114 disposed on the delivery tube 110 can self-expand. The distal expandable element 114 can self-expand such that the distal expandable element 114 circumferentially apposes walls of the blood vessel BV. The distal expandable element 114 can be porous having pores large enough to allow blood to flow therethrough while and small enough to capture fragments dislodged from the lesion P while the distal expandable element 114 is expanded as illustrated.

In FIG. 2C, the sheath 106 can be further retracted in the proximal direction 12, exposing the stent 118 disposed on the delivery tube 110. As the sheath 106 is retracted, the stent 118 can self-expand. The stent 118 can self-expand such that the stent 118 presses against the lesion P.

In FIG. 2D, the sheath 106 can be further retracted in the proximal direction 12, exposing the proximal expandable element 112. As the sheath 106 is retracted, the proximal expandable element 112 can self-expand. The proximal expandable element 112 can self-expand such that the element 112 circumferentially apposes walls of the blood vessel BV. As the stent 118 is expanded across the lesion P, fragments F from the lesion P can be dislodged. The proximal expandable element 112 and the distal expandable element 114 can prevent fragments F from the lesion P from migrating away from the lesion P.

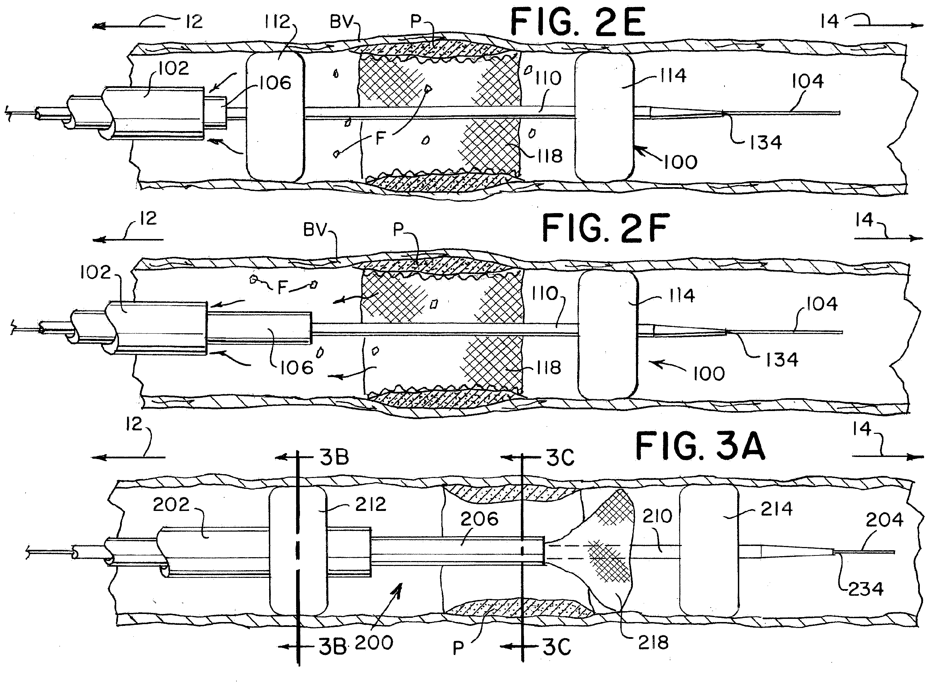

FIG. 2E illustrates aspiration through a lumen of the catheter 102. The proximal expandable element 112 can inhibit or allow blood flow. In examples where the proximal expandable element 112 inhibits blood flow, aspiration applied by the catheter 102 does not affect the fragments F while the proximal expandable element 112 is expanded. In examples where the proximal expandable element 112 allows blood flow, the proximal expandable element can include pores large enough to allow blood flow and small enough to capture fragments F. When aspiration is applied to a system including a porous proximal expandable element 112, aspiration can draw the fragments F toward the proximal expandable element 112.

FIG. 2F illustrates aspiration through the lumen of the catheter 102 while the sheath 106 is moved in the distal direction 14 to collapse the proximal expandable element 112. The lumens of the catheter 102 and the delivery tube 110 can be dimensioned to provide passage for fragments F to enter the catheter 102 while the delivery tube 110 and sheath 106 are extended therethrough.

FIG. 3A is another example of an intravascular treatment system 200 during treatment of a lesion P in a blood vessel BV. The treatment system 200 can include a delivery tube 210 sized to traverse a balloon guide catheter 202, a distal expandable element 214, a proximal balloon 212, a stent 218, and a sheath 206. The system 200 can be sized to traverse vasculature to lesion P. To reach the position illustrated in FIG. 3A, first a guide wire 204 can be extended through vasculature of the patient and positioned across the lesion P, then the balloon guide catheter 202 and the delivery tube 110 can be translated in the distal direction 14 over the guide wire 104 to the illustrated position.

The distal expandable element 214 can be disposed on the delivery tube 210 near a distal end 234 of the delivery tube 210. The proximal balloon 212 can be disposed on the balloon guide catheter 202 in the proximal direction 12 in relation to the distal expandable element 214. The stent 218 can be disposed over the delivery tube 210 in the proximal direction 12 in relation to the distal expandable element 214. During delivery of the system 200, the sheath 206 can be positioned over the stent 218 and distal expandable element 214 in order to prohibit the stent 218 and distal expandable element 214 from self-expanding.

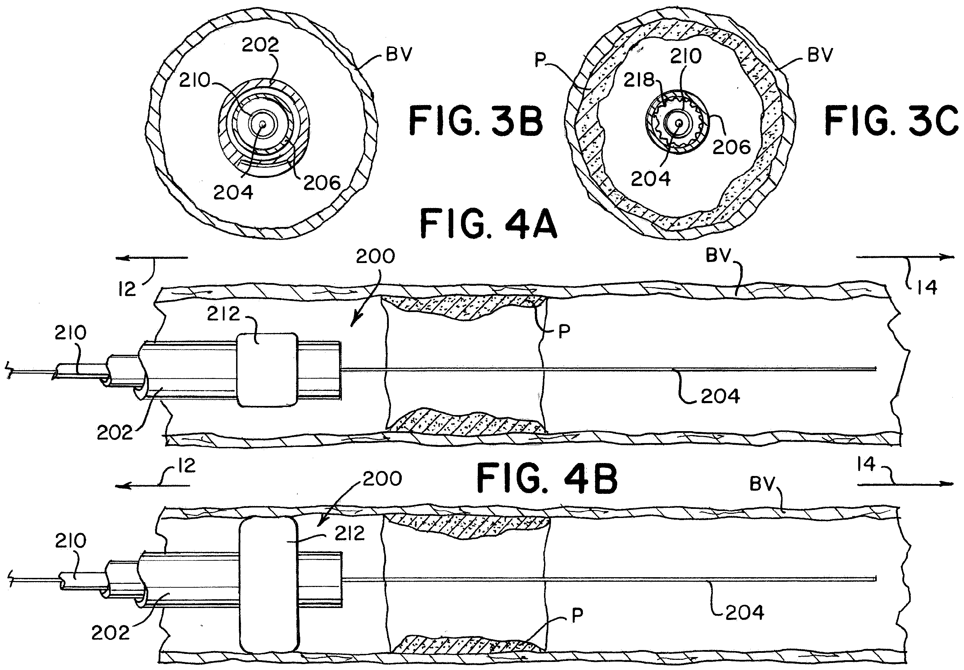

FIGs. 3B and 3C illustrate cross-sections of the intravascular treatment system 200 within a blood vessel BV as indicated in FIG. 3A. FIG. 3B illustrates a cross-section view of the balloon guide catheter 202 proximal to the proximal balloon 212. FIG. 3C illustrates a cross-section view of a proximal portion of the intravascular treatment system 200 relative to the stent 218 when positioned at the lesion P. In FIGs. 3B and 3C, the delivery tube 210 is delivered over the guide wire 204. FIG. 3B illustrates the delivery tube 210 surrounded by the sheath 206 can be disposed within the balloon guide catheter 202 during delivery. In FIG. 3C, the stent 218 can disposed over the delivery tube 210. The sheath 206 can surround the stent 218 to prevent the stent 218 from self-expanding.

FIGs. 4A through 4N illustrate a sequence of treatment steps using system 200. In FIG. 4A a guide wire 204 can be extended through vasculature BV of the patient and positioned across the lesion P. Then the balloon guide catheter 202 including the proximal balloon 212 disposed thereon can be positioned within the vasculature at a location proximal of the lesion P.

FIG. 4B illustrates the proximal balloon 212 can be inflated prior to the delivery tube 210 crossing the lesion P. The proximal balloon 212 can create a fluid impermeable blockage in the blood vessel BV when inflated. Inflating the proximal balloon 212 first can be particularly advantageous when the distal expandable element 214 is translatable to cross the lesion P after the proximal balloon 212 is expanded to thereby inhibit fragments dislodged during crossing of the lesion P from being carried elsewhere in the vasculature by blood flow.

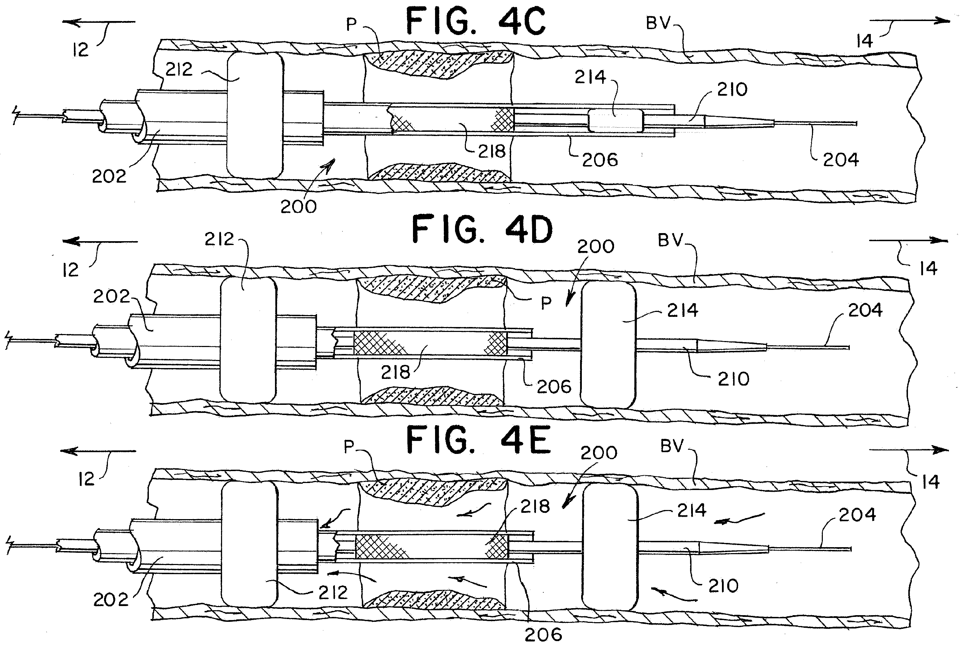

FIG. 4C illustrates the delivery tube 210 can be extended over the guide wire 204 through the vasculature such that the delivery tube 210 including the distal expandable element 214 and the stent 218 thereon can cross the lesion P. The sheath 206 can surround the stent 218 and the distal expandable element 214 to prevent the stent 218 and the distal expandable element 214 from expanding.

FIG. 4D illustrates the distal expandable element 214 can self-expand when the sheath 206 is retracted in the proximal direction 12. The distal expandable element 214 can expand to circumferentially appose the walls of the blood vessel BV. The distal expandable element 214 can be porous or fluid impermeable as described in relation to the distal expandable element 114 illustrated in FIGs. 1 and 2A through 2F.

FIG. 4E illustrates aspiration into the catheter 202. A passage of blood through the distal expandable element 214 can occur when the distal expandable element 214 is made of porous material. The pores of the distal expandable element 214 can be sized to inhibit dislodged fragments F from the lesion P from passing through the distal expandable element 214. The pores of the distal expandable element 214 can further be sized to allow the passage of blood as illustrated in FIG. 2E.

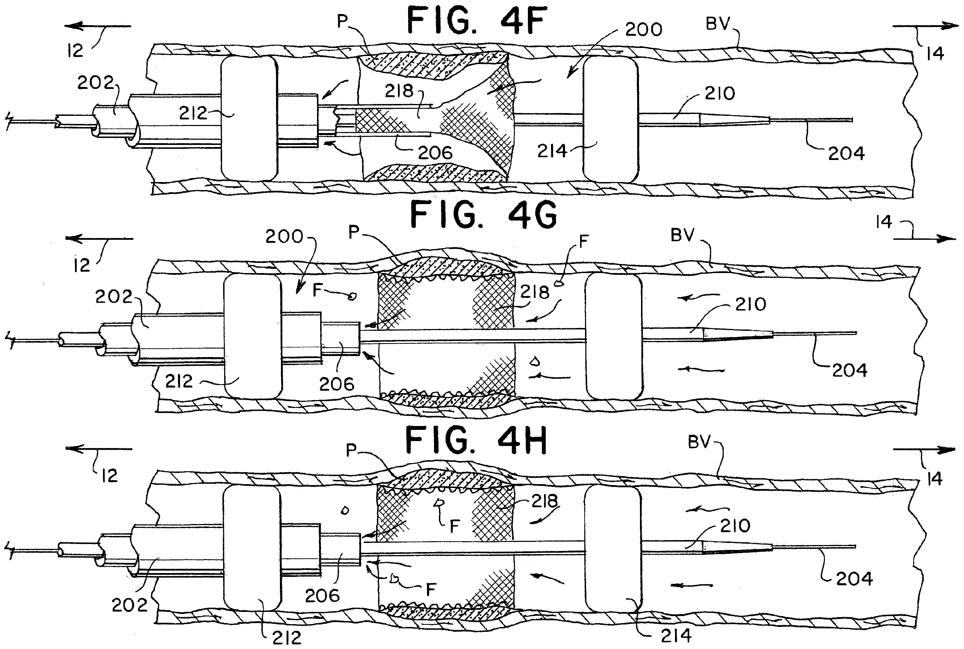

FIG. 4F illustrates the stent 218 can be capable of self-expanding when the sheath 206 is further retracted in the proximal direction 12. Aspiration can continue into the catheter 202 with blood flow as generally indicated by the arrows.

FIGs. 4G and 4H illustrate the stent 218 can self-expand such that the stent 218 presses against the lesion P. As the stent 218 presses against the lesion P, the walls of the blood vessel BV can additionally expand. Fragments F of the lesion P can become dislodged when the stent 218 presses against the lesion P. Fragments F dislodged during stenting can be aspirated through the balloon guide catheter 202. The expanded distal expandable element 214 and proximal balloon 212 can prevent fragments F from migrating away from the lesion and thus ensure that the fragment F are removed during aspiration.

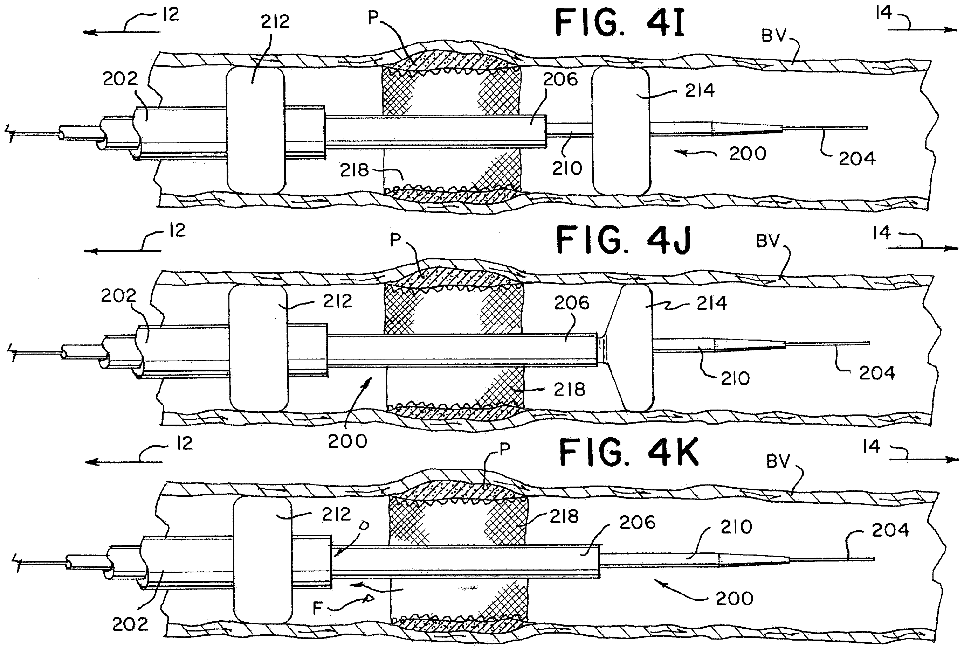

FIG. 4I illustrates the sheath 206 translating proximally through the stent 218 while the proximal balloon 212 and distal expandable element 214 remain expanded.

FIG. 4J illustrates the sheath 206 further translated distally to begin collapsing the distal expandable element 214 prior to deflating the proximal balloon 212. The sheath 206 can be moved further in the distal direction 14 to fully collapse the distal expandable element 214, as illustrated in FIG. 4K.

FIG. 4K further illustrates that sustained aspiration through the balloon guide catheter 202 can continue to remove fragments F from the blood vessel BV by allowing the fragments F to enter the lumen of the balloon guide catheter 202.

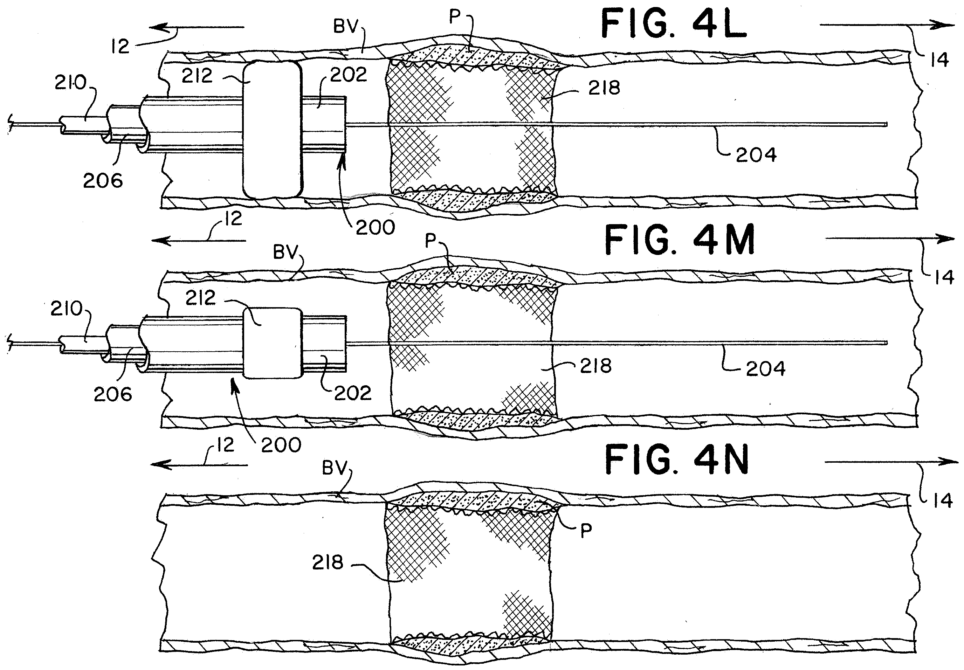

FIG. 4L illustrates the delivery tube 210 including the sheath 206 and distal expandable element (constricted by sheath 206) can be retracted into the lumen of the balloon guide catheter 202. The stent 218 can remain expanded across the lesion P.

FIG. 4M illustrates the proximal balloon 212 can be deflated. The stent 218 can remain expanded across the lesion P.

FIG. 4N illustrates the delivery tube 210 including the proximal balloon 212 and the guide wire 204 can be retracted from the blood vessel BV. The stent 218 can remain expanded across the lesion P.

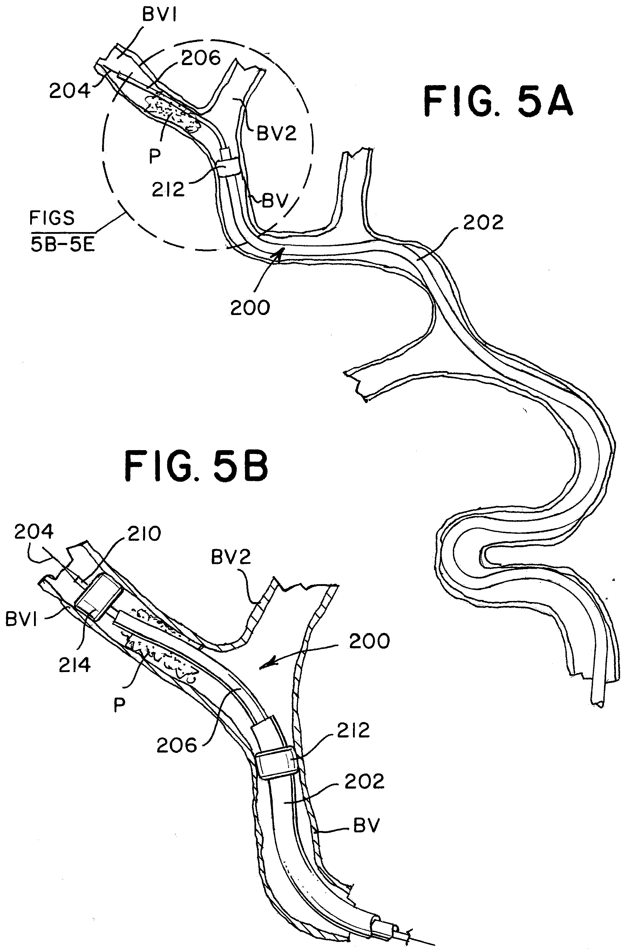

FIGs. 5A through 5E are a sequence of illustrations depicting steps of treatment of an intravascular lesion P at a bifurcation using system 200 as illustrated in FIG. 3. The bifurcation includes a stem blood vessel BV, a first branch blood vessel BV1, and a second branch blood vessel BV2. The lesion P is positioned in the first branch blood vessel BV1 in a distal direction 14 in relation to the opening to the second branch blood vessel BV2.

FIG. 5A illustrates translating system 200 through the vasculature to a location proximate the lesion P using the guide wire 204 and the balloon guide catheter 202. Further, FIG. 5A illustrates the delivery tube 210 extended across the lesion P with the proximal balloon 212 expanded to appose walls of the stem blood vessel BV in a proximal direction 12 in relation to the bifurcation between the first and second branch blood vessels BV1, BV2. The sheath 206 surrounds the delivery tube 210, preventing the distal expandable element 214 and the stent 218 from expanding.

FIG. 5B illustrates the sheath 206 can be retracted to allow the distal expandable element 214 disposed on the delivery tube 210 to self-expand. The distal expandable element 214 can be expanded such that the element 214 circumferentially apposes the walls of the first branch blood vessel BV1. In the expanded configuration, the distal expandable element 214 can occlude the first branch blood vessel BV1 in a distal direction 14 in relation to the lesion P.

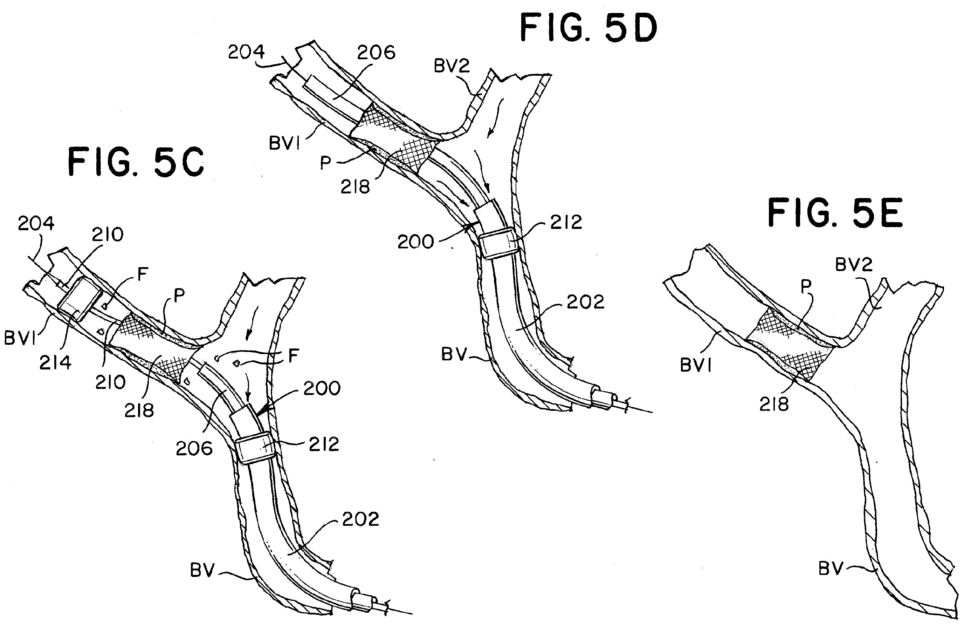

FIG. 5C illustrates the sheath 206 can be further retracted to allow the stent 218 to self-expand. As the stent 218 expands, the stent 218 can press into the lesion P. When the stent 218 presses into the lesion P, fragments F of the lesion P can become dislodged. The inflated proximal balloon 212 can be effective to arrest blood flow through the stem vessel BV. The distal expandable element 214 can be effective to inhibit fragments F dislodged from the lesion P from travelling in a distal direction 14 through the first branch blood vessel BV1. Aspiration through the lumen of the balloon guide catheter 202 while the distal expandable element 214 and the proximal balloon 212 remain expanded can occur. The lumen of the balloon guide catheter 202 can be sized, positioned, and otherwise configured to allow passage of the fragments F into the lumen of the balloon guide catheter 202 while the delivery tube 210 is in place.

FIG. 5D illustrates the distal expandable element 214 can be collapsed while the stent 218 remains expanded against the lesion P. The distal expandable element 214 can be collapsed by moving the sheath 206 over the distal expandable element 214. Aspiration through the lumen of the balloon guide catheter 202 can continue to remove the fragments F.

FIG. 5E illustrates the delivery tube 210 including the sheath 206 can be retracted into the lumen of the balloon guide catheter 202. The balloon guide catheter 202 can subsequently be retracted from the patient. The stent 218 can remain expanded across the first branch blood vessel BV1.

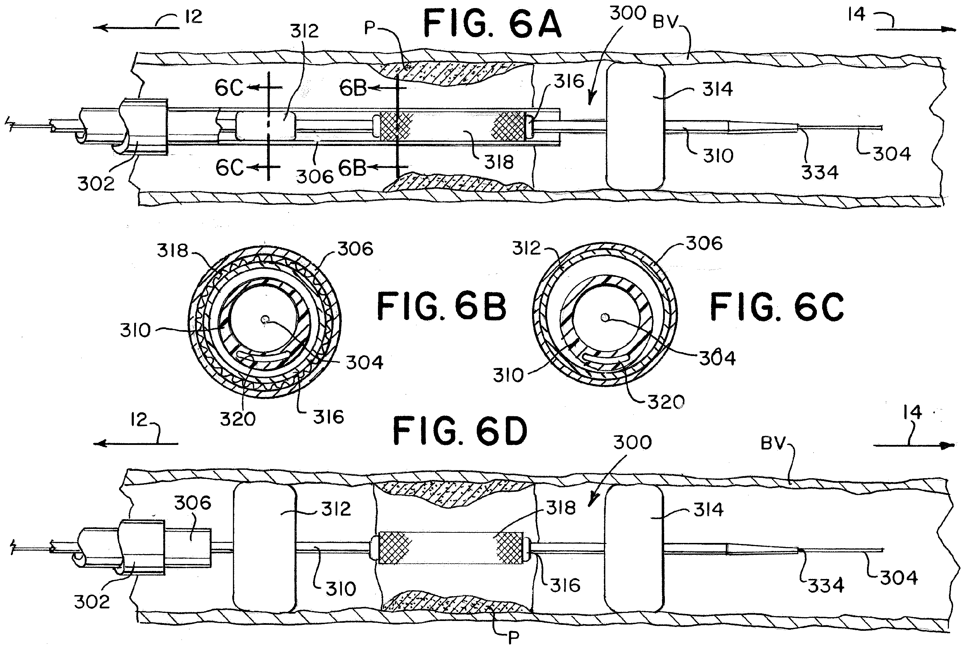

FIGs. 6A through 6F are a sequence of illustrations depicting steps of treatment using an alternative treatment system 300. FIG. 6A illustrates a delivery tube 310 positioned across the lesion P within a catheter 302. To reach the position illustrated in FIG. 6A, a guide wire 304 can be extended through vasculature of the patient and positioned across the lesion P, then the catheter 302 and delivery tube 310 can be translated in the distal direction 14 over the guide wire 304 to the illustrated position.

The delivery tube 310 can include a distal expandable element 314 disposed on the distal end 334 of the delivery tube 310. The distal expandable element 314 can be expanded to circumferentially appose the walls of the blood vessel BV, as illustrated in FIG. 6A. The distal expandable element 314 can be self-expandable. In this configuration, a sheath 306 can surround the distal expandable element 314 upon delivery to the desired location and subsequently retracted to allow the distal expandable element 314 to self-expand. Alternatively, the distal expandable element 314 can comprise a balloon that can be inflated.

The delivery tube 310 can further include a proximal expandable element 312 disposed in a proximal direction 12 from the distal expandable element 314. An angioplasty balloon 316 and stent 318 can be disposed over the delivery tube 310 between the proximal expandable element 312 and the distal expandable element 314. When the system 300 is being positioned across the lesion P, the sheath 306 surrounding the delivery tube 310 can prevent the proximal expandable element 312 from self-expanding.

FIGs. 6B and 6C illustrate cross-sections of the intravascular treatment system 300 as indicated in FIG. 6A. FIG. 6B illustrates a cross-section view of the intravascular treatment system 300 at the stent 318. FIG. 6C illustrates a cross-section view of a proximal portion of the intravascular treatment system 300 relative to the stent 318. In FIGs. 6B and 6C, the delivery tube 310 can be delivered over the guide wire 304. The delivery tube 310 can include an inflation lumen 320 to inflate angioplasty balloon 318. In FIG. 6B, the stent 318 is positioned over the angioplasty balloon 318, such that when the angioplasty balloon is inflated, the stent 318 can expand. The sheath 306 can surround the angioplasty balloon 318 prior to inflation. In FIG. 6C, the sheath 306 can surround the proximal expandable element 312 to prevent the proximal expandable element 312 from self-expanding.

FIG. 6D illustrates the sheath 306 can be retracted to allow the proximal expandable element 312 to self-expand. The proximal expandable element 312 can expand such that the element 312 circumferentially apposes the walls of the blood vessel BV.

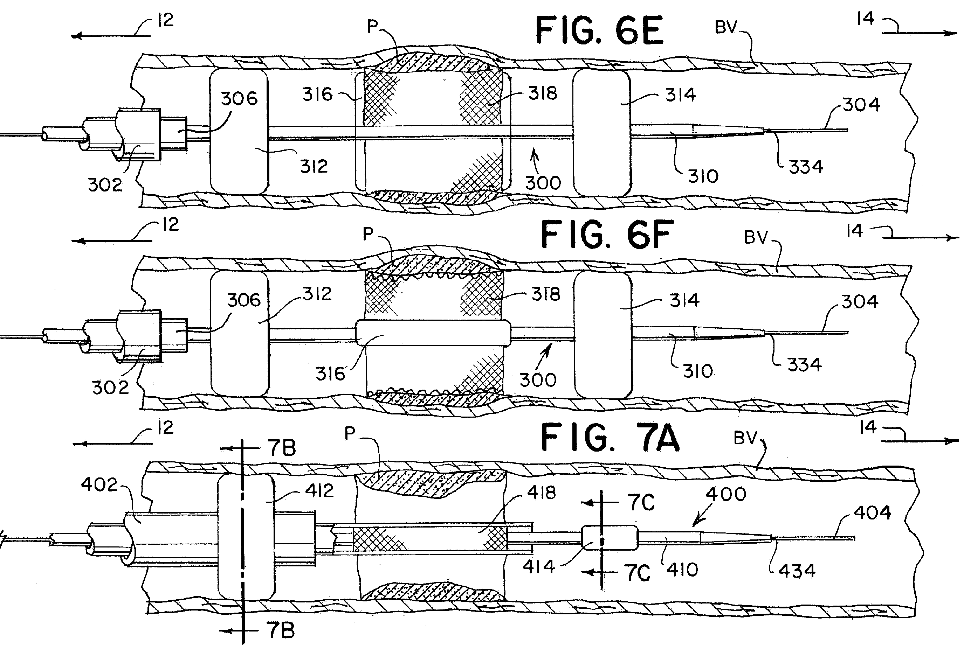

FIG. 6E illustrates the angioplasty balloon 316 can be inflated. Inflation of the angioplasty balloon 316 can cause the stent 318 to expand. Inflation of the angioplasty balloon 316 can press the stent 318 into the lesion P. Inflation of the angioplasty balloon 316 can additionally expand the blood vessel, as illustrated in FIGs. 6E and 6F.

In some treatments it can be advantageous to expand or inflate the distal expandable element 314 first to block blood flow downstream of the lesion P, retract the sheath 306 to expand the proximal expandable element 312 second to isolate fragments which may dislodge from the lesion P when the stent 318 is expanded, and inflate the angioplasty balloon 316 third, to expand the stent 318. When the distal expandable element 314 is expanded while the proximal expandable element 312 is collapsed, the distal expandable element 314 can anchor the delivery tube 310 in place and block the blood vessel BV while the system 300 still provides access for other treatment devices to reach the lesion P from the proximal direction 12.

FIG. 6F illustrates the angioplasty balloon 316 can be deflated. The distal expandable element 314 and the proximal expandable element 312 can remain expanded while the angioplasty balloon 316 is deflated. The stent 318 can remain expanded upon deflation of the angioplasty balloon 316. Inflation and deflation of the angioplasty balloon 316 can dislodge fragments F from the lesion P. The fragments F can be contained between the expanded distal element 314 and the proximal expandable element 312 without an aspiration force being applied. Aspiration can be applied immediately prior to advancing the sheath 306 in the distal direction 14 to collapse the proximal expandable element 312. By reducing the time and amount of aspiration, the volume of blood aspirated can be minimized.

Further treatment of the lesion P can continue as illustrated in FIGs. 2D through 2F.

FIGs. 7A through 7H are a sequence of illustrations depicting steps of treatment using an alternative treatment system 400. FIG. 7A illustrates a delivery tube 410 positioned across the lesion P within a catheter 402 having an expandable element 412 thereon. To reach the position illustrated in FIG. 7A, a guide wire 404 can be extended through vasculature of the patient and positioned across the lesion P, then the catheter 402 and delivery tube 410 can be translated in the distal direction 14 over the guide wire 404 to the illustrated position.

The delivery tube 410 can include a distal balloon 414 disposed at the distal end 434 of the delivery tube 410. The delivery tube 410 can further include a stent 418 that can be surrounded by a sheath 406.

The catheter 402 can include a proximal expandable element 412. The proximal expandable element 412 can be a permeable structure allowing blood and fluids to flow therethrough when expanded in the blood vessel BV and having pores small enough to inhibit liberated plaque fragments F from crossing the expanded proximal expandable element 412. The proximal expandable element 412 can be expanded prior to inflating the distal balloon 412 or the expanding the stent 418. Alternatively, the proximal expandable element 412 can be fluid impermeable when expanded such as a balloon.

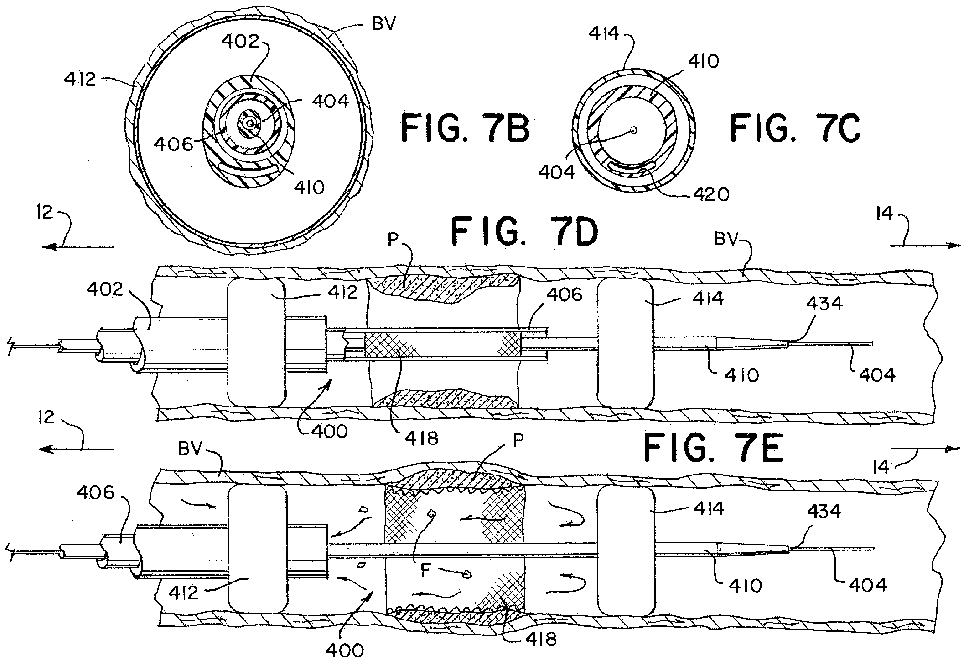

FIGs. 7B and 7C illustrate cross-sections of the intravascular treatment system 400 disposed within the blood vessel BV as indicated in FIG. 7A. FIG. 7B illustrates a cross-section view of the intravascular treatment system 400 at the proximal expandable element 412. FIG. 7C illustrates a cross-section view of the intravascular treatment system 400 at the distal balloon 414. In FIGs. 7B and 7C, the delivery tube 410 can be delivered over the guide wire 404. FIG. 7B illustrates the delivery tube 410 surrounded by the sheath 406 can be disposed within the catheter 402 during delivery. FIG. 7C illustrates the delivery tube 410 can include an inflation lumen 420 to inflate the distal balloon 414 disposed over the delivery tube 410.

FIG. 7D illustrates the distal balloon 414 can be inflated after the proximal expandable element 412 is expanded. The distal balloon 414 and the proximal expandable element 412 can be expanded such that the distal balloon 414 and the proximal expandable element 412 circumferentially appose the walls of the blood vessel. While the distal balloon 414 and proximal expandable element 412 are expanded, the sheath 406 can continue to surround the stent 418 to prevent the stent from self-expanding.

FIG. 7E illustrates the sheath 406 can be retracted into the lumen of the catheter 402, allowing the stent 418 to self-expand. As the stent 418 presses into the lesion P, fragments F of the lesion P can become dislodged. The fragments F can be contained between the expanded distal balloon 414 and the proximal expandable element 412.

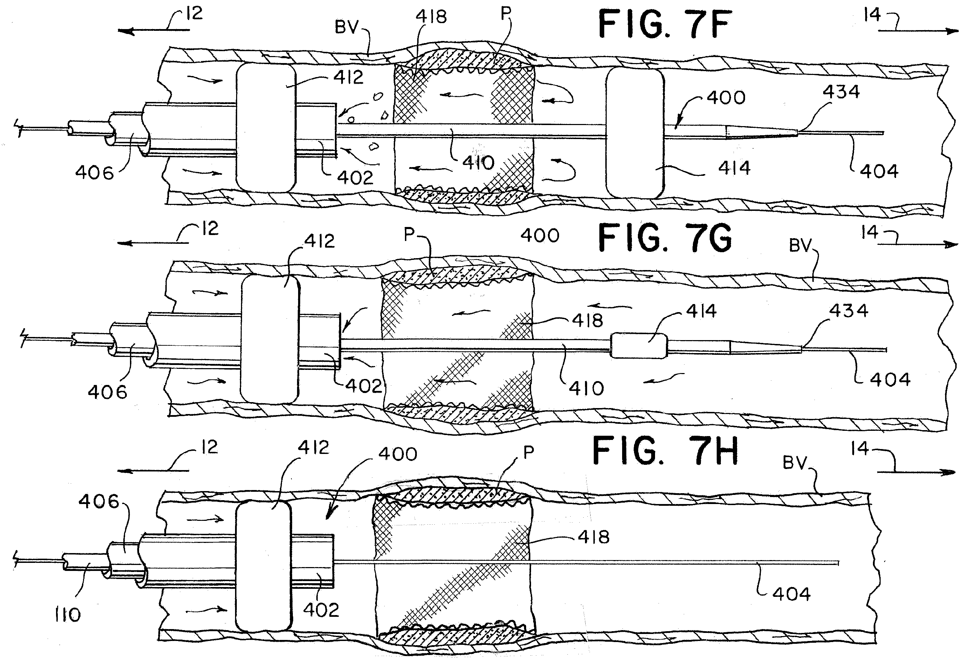

As illustrated in FIGs. 7E and 7F, when the proximal expandable element 412 is made of porous material, blood can pass through the proximal expandable element. However, the distal balloon 414 can inhibit blood from continuing to pass through the vasculature.

FIG. 7F illustrates aspiration through a lumen of the catheter 402 while the distal balloon 414 and the proximal expandable element 412 remain expanded in the blood vessel BV. The lumen of the catheter 402 can be sized, positioned, and otherwise configured to allow passage of the fragments F into the lumen of the catheter 402.

FIG. 7G illustrates the distal balloon 414 can be deflated. Aspiration can continue to allow the fragments F to be removed from the blood vessel BV. Further, when the distal balloon 414 is deflated, the passage of blood through porous proximal expandable element 412 can be maintained as the porous element 412 allows for blood flow.

FIG. 7H illustrates the delivery tube 410 including the distal balloon 414 can be retracted into the lumen of the catheter 402. The stent 418 can remain expanded across the lesion P.

Following retraction of the delivery tube 410, the proximal expandable element 412 can be collapsed and the catheter 402 can be retracted, leaving the stent 418 in place in the blood vessel BV.

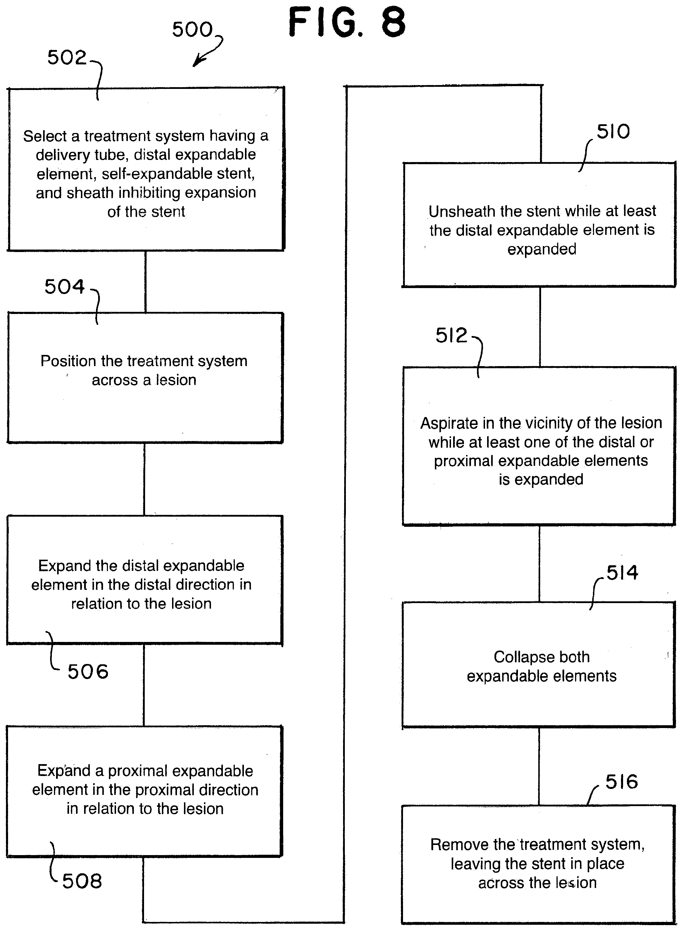

FIG. 8 is a flow diagram of a method 500 for treating an intravascular lesion. In step 502, a treatment system having a delivery tube, distal expandable element, self-expanding stent, and sheath inhibiting expansion of the stent can be selected. The delivery tube can be delivery tube 110, 210, 410 as illustrated and described herein, a variation thereof, or an alternative thereto as appreciated and understood by a person of ordinary skill in the art according to the teachings herein. The stent can be stent 118, 218, 418 as illustrated and described herein, a variation thereof, or an alternative thereto as appreciated and understood by a person of ordinary skill in the art according to the teachings herein.

In step 504, the treatment system can be positioned across a lesion.

In step 506, the distal expandable element, while disposed in the distal direction in relation to the lesion, can be expanded.

In step 508, the proximal expandable element, while disposed in the proximal direction in relation to the lesion, can be expanded.

In step 510, while at least the distal expandable element is expanded, the stent can be unsheathed.

In step 512, while at least one of the distal expandable element or proximal expandable elements is expanded, aspiration in the vicinity of the lesion can occur.

In step 514, the distal expandable element and the proximal expandable element can be collapsed.

In step 516, the treatment system can be removed while the stent can remain in place across the lesion in the blood vessel.

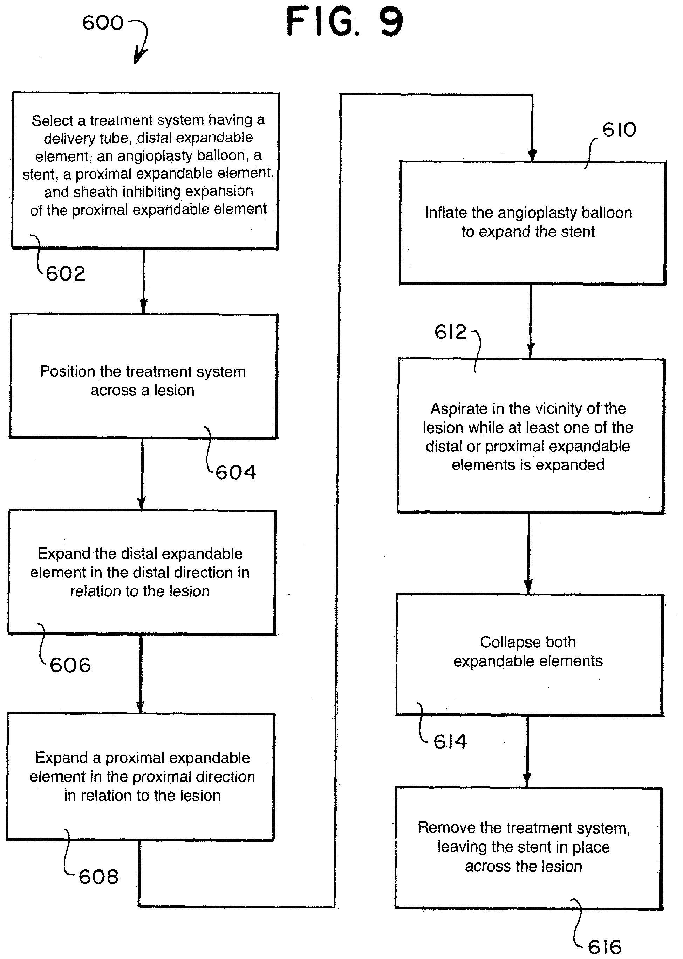

FIG. 9 is a flow diagram of an additional method 600 for treating an intravascular lesion. In step 602, a treatment system having a delivery tube, distal expandable element, an angioplasty balloon, a stent, a proximal expandable element, and sheath inhibiting expansion of the proximal expandable element can be selected. The delivery tube can be delivery tube 310 as illustrated and described herein, a variation thereof, or an alternative thereto as appreciated and understood by a person of ordinary skill int eh art according to the teaching herein. The stent can be stent 318 as illustrated and described herein, a variation thereof, or an alternative thereto as appreciated and understood by a person of ordinary skill in the art according to the teachings herein.

In step 604, the treatment system can be positioned across a lesion in the blood vessel.

In step 606, the distal expandable element, while disposed in the distal direction in relation to the lesion, can be expanded.

In step 608, the proximal expandable element, while disposed in the proximal direction in relation to the lesion, can be expanded.

In step 610, the angioplasty balloon can be inflated to expand the stent.

In step 612, while at least one of the distal expandable element or proximal expandable elements is expanded, aspirating in the vicinity of the lesion can occur.

In step 614, the distal expandable element and the proximal expandable element can be collapsed.

In step 616, the treatment system can be removed while the stent can remain in place across the lesion in the blood vessel.

The descriptions contained herein are examples of embodiments of the invention and are not intended in any way to limit the scope of the invention. As described herein, the invention contemplates many variations and modifications of the intravascular treatment system, including alternative materials, alternative device structures, alternative treatment steps, etc. Modifications apparent to those having ordinary skill in the art to which this invention relates and are intended to be within the scope of the claims which follow.

### ASPECTS OF THE INVENTION

1. A method for treating an intravascular lesion, the method comprising:
   selecting a treatment system comprising a delivery tube, distal expandable element, self-expandable stent, and sheath such that the distal expandable element and stent are disposed on the delivery tube and the sheath is disposed over the stent, the sheath positioned to inhibit the stent from expanding;
   positioning the treatment system across the lesion such that the distal expandable element is in the distal direction in relation to the lesion and the stent crosses the lesion;
   expanding the distal expandable element; and
   moving the sheath, thereby allowing the stent to expand into the lesion.
2. The method of aspect 1, wherein selecting the treatment system further comprises:
   selecting a balloon guide catheter comprising an expandable element thereon and lumen therethrough, the lumen sized to accommodate the delivery tube, the distal expandable element, the self-expanding stent, and the sheath, the method further comprising:
   delivering the delivery tube through vasculature, within the balloon guide catheter;
   positioning the proximal expandable element in a proximal direction in relation to the lesion; and
   expanding the proximal expandable element to circumferentially appose a blood vessel wall.
3. The method of aspect 2, further comprising:
   aspirating through the lumen of the guide catheter.
4. The method of aspect 2, wherein method steps are performed in the following order:
   expanding the distal expandable element;
   expanding the proximal expandable element; and
   moving the sheath, thereby allowing the stent to expand into the lesion.
5. The method of aspect 2, wherein method steps are performed in the following order:
   expanding the proximal expandable element;
   expanding the distal expandable element; and
   moving the sheath, thereby allowing the stent to expand into the lesion.
6. The method of aspect 1,
   wherein selecting a treatment system further comprises selecting the treatment system such that the sheath is disposed over the distal expandable element, the distal expandable element is self-expandable, and the sheath positioned to inhibit the distal expandable element from expanding,
   wherein expanding the distal expandable element further comprises moving the sheath to allow the distal expandable element to expand.
7. The method of aspect 6, further comprising:
   moving the sheath over the distal expandable element to collapse the distal occluding element.
8. The method of aspect 1, wherein expanding the distal expandable element further comprises inflating the distal expandable element.

## Claims

1. An intravascular treatment system comprising:
a delivery tube sized to traverse vasculature;
a distal expandable element disposed on the delivery tube and approximate a distal end of the delivery tube;
a self-expandable stent disposed over a portion of the delivery tube in a proximal direction in relation to the distal expandable element; and
a sheath surrounding the self-expandable stent, positioned to inhibit the self-expandable stent from expanding, and movable to allow the self-expandable stent to expand.

2. The intravascular treatment system of claim 1, further comprising:
a guide catheter comprising a lumen therethrough sized to accommodate the delivery tube, the distal expandable element, the self-expanding stent, and the sheath.

3. The intravascular treatment system of claim 2, wherein the lumen of the guide catheter is sufficiently contiguous to be suitable for aspiration within vasculature.

4. The intravascular treatment system of claim 1, wherein the distal expandable element comprises a balloon.

5. The intravascular treatment system of claim 1,
wherein the distal expandable element comprises a self-expandable portion, wherein the sheath surrounds the distal expandable element,
wherein the sheath is positioned to inhibit the distal expandable element from expanding, and
wherein the sheath is movable to allow the distal expandable element to expand.

6. The intravascular treatment system of claim 1,
wherein the distal expandable element is porous.

7. The intravascular treatment system of claim 1, further comprising:
a proximal expandable element disposed on the delivery tube in the proximal direction in relation to the stent.

8. The intravascular treatment system of claim 7, wherein the proximal expandable element is porous.

9. The intravascular treatment system of claim 7, further comprising:
an aspiration catheter comprising a lumen therethrough sized to receive the delivery tube.

10. An intravascular treatment system comprising:
a delivery tube sized to traverse vasculature;
an expandable distal occluding element disposed on the delivery tube and approximate a distal end of the delivery tube;
an angioplasty balloon disposed over the delivery tube in a proximal direction in relation to the distal occluding element;
a stent disposed over the angioplasty balloon;
a self-expandable proximal occluding element disposed on the delivery tube in the proximal direction in relation to the stent; and
a sheath surrounding the stent and the proximal occluding element, the sheath being positioned to inhibit the proximal occluding element from expanding, and the sheath being movable to allow the proximal occluding element to expand.

11. The intravascular treatment system of claim 10, wherein the sheath is movable to collapse the proximal occluding element after being moved to expand the proximal occluding element.

12. The intravascular treatment system of claim 10, wherein the proximal occluding element is porous.
